# FASCICULE DE BREVET EUROPEEN

(11) **EP 1 372 825 B1**
(45) Date de publication et mention de la délivrance du brevet: **12.03.2008**
(21) Numéro de dépôt: 02759744.2
(22) Date de dépôt: 27.03.2002
(51) Int. Cl.: B01D 53/68, B01J 20/08, C07C 17/38

(54) **PROCEDE D'ELIMINATION DES HALOGENURES METALLIQUES PRESENTS DANS UN EFFLUENT ORGANIQUE OU NON ORGANIQUE, LIQUIDE OU GAZEUX**
VERFAHREN ZUR BESEITIGUNG VON IN ORGANISCHEN ODER ANORGANISCHEN ABWÄSSERN ODER ABGASEN ENTHALTENEN METALLHALIDEN
METHOD FOR ELIMINATING METAL HALIDES THAT ARE PRESENT IN A LIQUID OR GASEOUS, ORGANIC OR NON-ORGANIC EFFLUENT

(30) Priorité: 04.04.2001 FR 0104590
(43) Date de publication de la demande: 02.01.2004
(73) Titulaire: AXENS, 92500 Rueil Malmaison (FR)
(72) Inventeur: NEDEZ, Christophe, F-30340 Salindres (FR)
(74) Mandataire: Benoist, François
(86) Numéro de dépôt international: PCT/FR2002/001070
(87) Numéro de publication internationale: WO 2002/081063

(56) Documents cités:
- FR-A- 2 774 606
- GB-A- 1 223 238
- US-A- 4 444 899
- US-A- 5 316 998
- PATENT ABSTRACTS OF JAPAN vol. 2000, no. 10, 17 novembre 2000 (2000-11-17) & JP 2000 203829 A (TAIMEI TAKAYUKI CO), 25 juillet 2000 (2000-07-25)

## Description

L'invention concerne le domaine de l'élimination des impuretés contenues dans des effluents industriels organiques ou non organiques à l'état liquide ou gazeux. Plus précisément, elle concerne l'élimination d'impuretés constituées par des halogénures métalliques contenus dans ces effluents, par adsorption sur des agglomérés d'alumine.

De nombreux effluents industriels gazeux ou liquides contiennent des impuretés dont l'élimination est désirable. Ces impuretés peuvent poser des problèmes d'ordres divers. Parmi ceux-ci on peut citer :
- la perturbation de procédés auxquels participe l'effluent, par formation de produits indésirables, ou inhibition ou empoisonnement d'un catalyseur ;
- la dégradation de la qualité du produit final résultant d'un défaut de pureté, d'une coloration parasite, etc ;
- la formation de rejets industriels issus d'un traitement de l'effluent difficiles à retraiter, et posant donc des problèmes environnementaux.

Il est connu d'améliorer la pureté de certains effluents industriels liquides ou gazeux, organiques ou non organiques, en les faisant passer sur un matériau minéral tel que l'alumine, qui retient certaines impuretés par adsorption à sa surface. Il est, en particulier, connu d'utiliser des agglomérés d'alumine pour épurer des effluents industriels contenant des halogénures métalliques à l'état de traces, que l'on désire éliminer. Il est habituellement admis que ces alumines doivent présenter une haute surface spécifique et donc des pores majoritairement de très petite taille.

Le but de l'invention est de proposer un procédé d'épuration d'effluents industriels contenant des halogénures métalliques par adsorption sélective sur de l'alumine, utilisant des formes d'alumine relativement peu coûteuses à fabriquer, et présentant néanmoins une excellente efficacité pour l'application envisagée, bien meilleure que celle des alumines utilisées à cet effet dans l'art antérieur.

A cet effet, l'invention a pour objet un procédé d'élimination des halogénures métalliques présents dans un effluent organique ou non organique, liquide ou gazeux, selon lequel on réalise cette élimination par adsorption desdits halogénures métalliques sur des agglomérés d'alumine, caractérisé en ce que :
- lesdits agglomérés ont une surface spécifique comprise entre 50 et 350 m²/g, préférentiellement comprise entre 70 et 300 m²/g, très préférentiellement comprise entre 80 et 250 m²/g ; et en ce que
- lesdits agglomérés ont un V_{80Å} supérieur ou égal à 20 ml/100g, préférentiellement supérieur ou égal à 25 ml/100g, très préférentiellement supérieur ou égal à 30ml/100g, optimalement supérieur ou égal à 35 ml/100g.

Lesdits agglomérés ont de préférence un V_{400Å} supérieur ou égal à 10 ml/100g, préférentiellement supérieur ou égal à 15 ml/100g, très préférentiellement supérieur ou égal à 20 ml/100g.

Lesdits agglomérés ont de préférence un V_{37Å} supérieur ou égal à 45ml/100g, préférentiellement supérieur ou égal à 55ml/100g.

Lesdits agglomérés peuvent comporter un ou des composés dopants sélectionnés parmi les composés de métaux alcalins, de métaux alcalino-terreux et de terres rares, à une teneur maximale de 20%, de préférence inférieure à 10%.

Lesdits agglomérés peuvent être sous forme de billes, ayant de préférence un diamètre inférieur ou égal à 8 mm, préférentiellement compris entre 1 et 5 mm.

Lesdits agglomérés peuvent se présenter sous forme de matériaux extrudés, par exemple de forme cylindrique ou polylobée.

Lesdits matériaux extrudés ont de préférence un diamètre inscrit de leur section inférieur ou égal à 4 mm.

Lesdits agglomérés d'alumine peuvent se présenter sous forme de poudre.

Dans une application particulière de l'invention, ledit effluent est trouvé en aval d'une production de polychlorure de vinyle. Le milieu peut alors être à base de dichloréthylène, et ledit halogénure métallique est du chlorure ferrique.

Comme on l'aura compris, l'invention consiste à utiliser comme matériaux adsorbants sélectifs une classe d'agglomérés d'alumine présentant des caractéristiques particulières à la fois en termes de surface spécifique et de structure poreuse. De manière surprenante eu égard à ce que l'on avait précédemment expérimenté, ces agglomérés doivent avoir une surface spécifique relativement réduite, et un profil de porosité où les pores de très petit diamètre ne représentent pas forcément un volume très important. Ces agglomérés possèdent cependant des propriétés adsorbantes remarquablement élevées vis à vis des halogénures métalliques renfermés par les effluents industriels, liquides ou gazeux, organiques ou non organiques (ces derniers pouvant être, par exemple, des solutions aqueuses).

L'invention sera mieux comprise à l'aide de la description qui suit, donnée en référence à la figure unique annexée. Celle-ci montre, pour différents agglomérés d'alumine selon l'invention et différents agglomérés d'alumine de référence, le taux de disparition (en %) du chlorure ferrique renfermé par une solution de chlorure ferrique dans de l'acétophénone mise au contact d'un aggloméré d'alumine, au bout de 37 heures de réaction.

L'invention trouve une application privilégiée, mais en aucun cas limitative, en aval d'une chaîne de production de polychlorure de vinyle (PVC). Après la polymérisation du PVC, il peut subsister un milieu résiduel à base de dichloréthylène qui contient des traces de chlorure ferrique. Ce chlorure ferrique doit être éliminé avant le recyclage de ce milieu résiduel. Il est déjà connu de réaliser cette élimination par adsorption sur des agglomérés d'alumine tels que ceux cités plus loin à titre de matériaux de référence. On va voir que l'utilisation, à cet effet, d'une classe particulière d'agglomérés d'alumine procure une efficacité sensiblement améliorée à une opération d'élimination du chlorure ferrique.

Les agglomérés d'alumine utilisés dans le cadre du procédé d'élimination des halogénures métalliques selon l'invention doivent avoir obligatoirement une surface spécifique comprise entre 50 et 350 m²/g, de préférence comprise entre 70 et 300 m²/g, avantageusement comprise entre 80 et 250 m²/g. De manière également obligatoire, ils ont un volume occupé par les pores de diamètre supérieur ou égal à 80 Å (désigné en abrégé par la notation V_{80Å}) supérieur ou égal à 20ml/100g, de préférence supérieur ou égal à 25ml/100g, avantageusement supérieur ou égal à 30ml/100g, voire supérieur ou égal à 35 ml/100g.

On notera que le degré de présence de pores de diamètre plus petit que 80Å n'a que peu d'importance dans le cadre de l'invention. La faible importance accordée à une telle microporosité va, comme on l'a dit, à l'encontre de ce qui était communément admis jusque là.

Selon une variante préférée de l'invention, le volume occupé par les pores de diamètre supérieur ou égal à 400Å (V_{400Å}) est supérieur ou égal à 10ml/100g, avantageusement supérieur ou égal à 20 ml/100g.

Le V_{80Å} et le V_{400Å} peuvent être déterminés par une méthode classique de porosimétrie au mercure.

A cet effet, l'échantillon d'alumine est placé dans une colonne, dans laquelle on introduit du mercure sous une pression P. Le mercure ne mouillant pas l'alumine, sa pénétration ou sa non-pénétration dans les pores de l'échantillon ayant un diamètre donné est fonction de la valeur de P. Les pores les plus fins nécessitent, pour être remplis, l'établissement d'une pression P plus élevée que pour le remplissage des pores plus grossiers. La mesure de la quantité de mercure pénétrant dans l'échantillon pour différentes valeurs de P permet de connaître le volume occupé par les pores de diamètre supérieur à des valeurs données de ce diamètre.

Selon une forme particulière de l'invention, les agglomérés d'alumine peuvent être chimiquement modifiés par l'apport de composés de métaux alcalins ou alcalino-terreux, ou de terres rares, ou d'un mélange de tels composés.

Préférentiellement, on choisit des composés à base de sodium, de potassium, de calcium, de magnésium ou de lanthane. Le sodium est un exemple privilégié, que l'on peut introduire sous forme d'un ou de plusieurs précurseurs de son oxyde Na₂O.

L'ajout du ou des composés dopants peut être effectué antérieurement ou postérieurement à l'opération de mise en forme, ou pendant celle-ci.

Les composés dopants sont présents dans l'aggloméré d'alumine à raison d'une teneur massique totale inférieure à 20%, de préférence inférieure à 10%.

Ces composés dopants permettent d'accentuer les propriétés adsorbantes de la surface des agglomérés d'alumine vis à vis des molécules d'halogénures métalliques dont l'élimination est recherchée.

L'alumine peut être utilisée sous forme de poudre, mais de préférence, elle est utilisée après une étape de mise en forme. Des billes, de diamètre avantageusement inférieur à 8 mm, de préférence majoritairement compris entre 1 et 5 mm, constituent une telle forme préférée des agglomérés d'alumine selon l'invention. Une autre forme préférée est celle d'extrudés cylindriques ou polylobés, dont le diamètre inscrit de leur section est de préférence inférieur à 4 mm.

Les billes peuvent être obtenues au moyen d'une technologie tournante, par agglomération d'une poudre d'alumine dans un drageoir ou un tambour. De manière connue, ce type de procédé permet d'obtenir des billes de diamètre et de répartitions de pores contrôlés, ces dimensions et ces répartitions étant, en général, créées pendant l'étape d'agglomération. La porosité peut être créée par différents moyens, comme le choix de la granulométrie de la poudre d'alumine ou l'agglomération de plusieurs poudres d'alumine de différentes granulométries. Une autre méthode consiste à mélanger à la poudre d'alumine, avant ou pendant l'étape d'agglomération, un composé, appelé porogène, disparaissant par chauffage et créant ainsi une porosité dans les billes. Comme composés porogènes utilisés, on peut citer, à titre d'exemple, la farine de bois, le charbon de bois, le soufre, des goudrons, des matières plastiques ou émulsions de matières plastiques telles que le polychlorure de vinyle, des alcools polyvinyliques, la naphtaline ou analogues. La quantité de composés porogènes ajoutés est déterminé par le volume désiré. Un ou plusieurs traitements thermiques viennent alors achever la mise en forme des billes.

Les extrudés pourront être obtenus par malaxage puis extrusion d'un gel d'alumine, ou d'une poudre d'alumine, ou d'un mélange de matières premières différentes.

La poudre d'alumine initiale peut être obtenue de manière classique par déshydratation rapide d'un hydroxyde d'aluminium (hydrargillite, par exemple).

L'ajout du ou des composés dopants peut être effectué antérieurement ou postérieurement à l'opération de mise en forme ou pendant celle-ci.

A titre d'exemples, on va à présent comparer les résultats d'adsorption du chlorure ferrique FeCl₃ pour divers agglomérés d'alumine de référence et pour des agglomérés d'alumine ayant les caractéristiques exigées par le procédé selon l'invention.

Huit agglomérés d'alumines ont été considérés, l'utilisation des alumines A, B et C faisant partie de l'art antérieur, et l'utilisation des alumines D, E, F, G et H correspondant à l'invention.

100 ppm de FeCl₃, 6H₂O sont placés dans un bécher contenant 250 ml d'acétophénone. Est alors ajouté 1 g d'alumine prétraitée à 300°C sous forme de billes ou de matériaux extrudés. Le bécher est alors isolé de l'air ambiant, protégé de la lumière pour éviter toute dégradation du solvant (photosensible) et placé sous agitation magnétique, les billes ou extrudés étant isolés du barreau aimanté afin d'éviter tout problème d'attrition parasite au cours de l'expérience.

Les agglomérés d'alumine mis en oeuvre sont décrits dans le Tableau I, où les diamètres sont donnés en mm, les surfaces spécifiques en m²/g, les porosités en ml/100g. Le Tableau I mentionne, outre les paramètres précédemment cités, le V_{37Å} de chaque aggloméré, c'est à dire le volume occupé par les pores de diamètre supérieur ou égal à 37Å. L'écart entre le V_{37Å} et le V_{80Å} est représentatif de la quantité de pores de très petits diamètres de l'aggloméré testé. On mentionne, enfin, la teneur en Na₂O des agglomérés, exprimée en ppm.

**Tableau I. Caractéristiques des agglomérés d'alumine testés**

| | Références | | | invention | | | | |
|---|---|---|---|---|---|---|---|---|
| Alumine | A | B | C | D | E | F | G | H |
| Forme | Billes | Billes | Billes | Extrudés | Billes | Billes | Extrudés | Billes |
| Diamètre | 1,4-2,8 | 1,4-2,8 | 2-4 | 1,2 | 2,0-2,8 | 1,4-2,8 | 1,2 | 2,0-2,8 |
| Surface spécifique | 337 | 257 | 6 | 266 | 196 | 150 | 251 | 172 |
| V_{37Å} | 35,6 | 37,2 | 53,5 | 68,4 | 68,0 | 102,3 | 59,8 | 58,4 |
| V_{80Å} | 14,1 | 9,4 | 53,4 | 48,9 | 57,5 | 98,5 | 41,5 | 46,5 |
| V_{400Å} | 5,3 | 4,6 | 53,2 | 5,8 | 20,0 | 53,7 | 5,4 | 15,2 |
| Na₂O | 3500 | 20000 | 700 | 500 | 700 | 700 | 20000 | 20000 |

Le suivi de la concentration de la solution d'acétophénone en chlorure de fer est effectué par analyse en UV-visible, en suivant en particulier l'évolution de l'absorbance à la longueur d'onde de 378 nm.

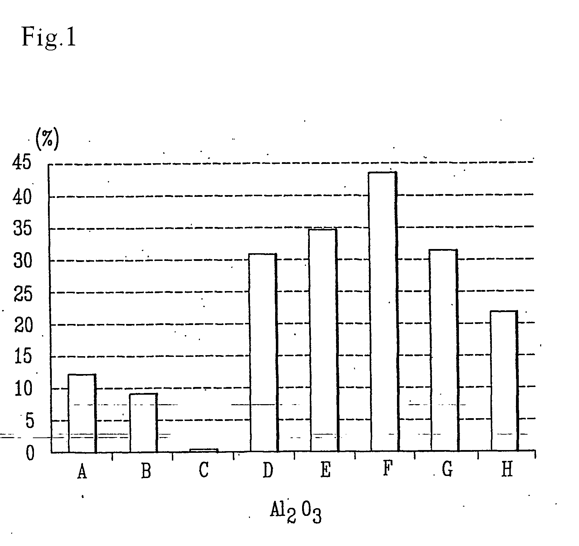

Au bout de 37 heures de réaction à température ambiante, les taux de disparition du chlorure de fer dans la solution organique ont été ainsi évalués, et sont reportés sur le diagramme de la figure 1.

Les alumine utilisées dans l'art antérieur montrent un potentiel d'adsorption nettement inférieur à celui des alumines utilisées dans le procédé selon l'invention.

L'alumine C de référence, qui ne comporte que des pores de diamètre élevé et une très faible surface spécifique, est d'une efficacité très médiocre pour l'adsorption du chlorure ferrique.

On notera que les alumines du procédé selon l'invention présentent une surface spécifique qui n'est pas particulièrement élevée : elle est du même ordre ou franchement inférieure à celle de l'alumine B de référence, et franchement inférieure à celle de l'alumine A de référence.

Par rapport aux alumines A et B de référence qui ont une surface spécifique comparable ou supérieure, les alumines selon l'invention se distinguent par leur V_{80Å} relativement élevé. Mais on note également que dans beaucoup de cas, les alumines selon l'invention présentent un écart entre le V_{80Å} et le V_{37Å} faible, ce qui est l'indice qu'elles n'ont que peu de pores de très petite taille. C'est, en particulier, le cas de l'alumine F qui présente les meilleurs résultats pour l'adsorption du chlorure ferrique. De manière toutefois préférée, le V_{37Å} sera utilement d'au moins 45ml/100g, préférentiellement supérieur à 55ml/100g. On notera également que cette alumine F a un V_{400Å} élevé, donc des pores de gros diamètre présents en assez forte quantité. Tout cela se traduit par une surface spécifique assez peu élevée, ce qui, néanmoins, ne compromet pas la qualité des résultats obtenus, bien au contraire. Ces résultats vont donc à l'encontre de ce qui était communément admis comme devant constituer les caractéristiques préférées des agglomérés d'alumine, dans leur application envisagée à l'adsorption des chlorures métalliques.

On notera enfin que les alumines des exemples selon l'invention n'ont pas des teneurs en Na₂O (composé dopant) très élevées : elles sont au maximum de 2%. Malgré cela, elles présentent des propriétés d'adsorption du chlorure ferrique sensiblement plus élevées que les alumines de référence dont la teneur en Na₂O est comparable. Cela montre bien que pour cette application, le rôle de la porosité des agglomérés est prépondérant, le dopage de ces agglomérés par des composés d'alcalins ou d'alcalino-terreux n'étant qu'une variante de l'invention.

L'invention n'est pas limitée aux exemples précis qui ont été cités, à savoir l'adsorption du chlorure ferrique d'un effluent à base de dichloréthylène ou d'acétophénone. L'utilisation d'agglomérés d'alumine tels que décrits pour l'adsorption d'halogénures métalliques peut être envisagée pour le traitement de tous effluents gazeux ou liquides, organiques ou non organiques. Elle est, en particulier, applicable aux solutions aqueuses.

## Revendications

1. Procédé d'élimination des halogénures métalliques présents dans un effluent organique ou non organique, liquide ou gazeux, selon lequel on réalise cette élimination par adsorption desdits halogénures métalliques sur des agglomérés d'alumine, **caractérisé en ce que** :
- lesdits agglomérés ont une surface spécifique comprise entre 50 et 350 m²/g, préférentiellement comprise entre 70 et 300 m²/g, très préférentiellement comprise entre 80 et 250 m²/g ; et **en ce que**
- lesdits agglomérés ont un V_{80Å} supérieur ou égal à 20 ml/100g, préférentiellement supérieur ou égal à 25 ml/100g, très préférentiellement supérieur ou égal à 30ml/100g, optimalement supérieur ou égal à 35 ml/100g.

2. Procédé selon la revendication 1, **caractérisé en ce que** lesdits agglomérés ont un V_{400Å} supérieur ou égal à 10 ml/100g, préférentiellement supérieur ou égal à 15 ml/100g, très préférentiellement supérieur ou égal à 20 ml/100g.

3. Procédé selon la revendication 1 ou 2, **caractérisé en ce que** lesdits agglomérés ont un V_{37Å} supérieur ou égal à 45ml/100g, préférentiellement supérieur ou égal à 55ml/100g.

4. Procédé selon l'une des revendications 1 à 3, **caractérisé en ce que** lesdits agglomérés comportent un ou des composés dopants sélectionnés parmi les composés de métaux alcalins, de métaux alcalino-terreux et de terres rares, à une teneur maximale de 20%, préférentiellement inférieure à 10%.

5. Procédé selon l'une des revendications 1 à 4, **caractérisé en ce que** lesdits agglomérés sont sous forme de billes.

6. Procédé selon la revendication 5, **caractérisé en ce que** lesdites billes ont un diamètre inférieur ou égal à 8 mm, préférentiellement compris entre 1 et 5 mm.

7. Procédé selon l'une des revendications 1 à 4, **caractérisé en ce que** lesdits agglomérés se présentent sous forme de matériaux extrudés.

8. Procédé selon la revendication 7, **caractérisé en ce que** lesdits matériaux extrudés ont une forme cylindrique.

9. Procédé selon la revendication 7, **caractérisé en ce que** lesdits matériaux extrudés ont une forme polylobée.

10. Procédé selon l'une des revendications 7 à 9, **caractérisé en ce que** lesdits matériaux extrudés ont un diamètre inscrit de leur section inférieur ou égal à 4 mm.

11. Procédé selon l'une des revendications 1 à 4, **caractérisé en ce que** lesdits agglomérés d'alumine se présentent sous forme de poudre.

12. Procédé selon l'une des revendications 1 à 11, **caractérisé en ce que** ledit effluent est un milieu à base de dichloréthylène, et **en ce que** ledit halogénure métallique est du chlorure ferrique.

## Claims

1. A method of eliminating metal halides that are present in a liquid or gaseous, organic or non-organic effluent, in which this elimination is carried out by adsorption of said metal halides on alumina agglomerates, **characterized in that**:
- said agglomerates have a specific surface area of between 50 and 350 m²/g, preferably between 70 and 300 m²/g and even more preferably between 80 and 250 m²/g; and **in that**
- said agglomerates have a V_{80Å} of greater than or equal to 20 ml/100 g, preferably greater than or equal to 25 ml/100 g, even more preferably greater than or equal to 30 ml/100 g and optimally greater than or equal to 35 ml/100 g.

2. The method as claimed in claim 1, **characterized in that** said agglomerates have a V_{400Å} of greater than or equal to 10 ml/100 g, preferably greater than or equal to 15 ml/100 g and even more preferably greater than or equal to 20 ml/100 g.

3. The method as claimed in claim 1 or 2, **characterized in that** said agglomerates have a V_{37Å} of greater than or equal to 45 ml/100 g and preferably greater than or equal to 55 ml/100 g.

4. The method as claimed in one of claims 1 to 3, **characterized in that** said agglomerates comprise one or more dopant compounds selected from compounds of alkali metals, alkaline-earth metals and rare earths, having a maximum content of 20%, preferably less than 10%.

5. The method as claimed in one of claims 1 to 4, **characterized in that** said agglomerates are in the form of beads.

6. The method as claimed in claim 5, **characterized in that** said beads have a diameter of less than or equal to 8 mm, preferably between 1 and 5 mm.

7. The method as claimed in one of claims 1 to 4, **characterized in that** said agglomerates are in the form of extruded materials.

8. The method as claimed in claim 7, **characterized in that** said extruded materials are of cylindrical shape.

9. The method as claimed in claim 7, **characterized in that** said extruded materials are of polylobate shape.

10. The method as claimed in one of claims 7 to 9, **characterized in that** said extruded materials have an inscribed diameter of their cross section of less than or equal to 4 mm.

11. The method as claimed in one of claims 1 to 4, **characterized in that** said alumina agglomerates are in the form of powder.

12. The method as claimed in one of claims 1 to 11, **characterized in that** said effluent is a dichloroethylene-based medium and **in that** said metal halide is ferric chloride.

## Patentansprüche

1. Verfahren zum Entfernen von Metallhalogeniden, die in einem organischen oder anorganischen Abwasser oder Abgas vorkommen, wobei das Entfernen durch Adsorption der Metallhalogenide auf Aluminiumoxid-Agglomeraten erfolgt, **dadurch gekennzeichnet, dass**:
- die Agglomerate eine spezifische Oberfläche aufweisen, die zwischen 50 und 350 m²/g liegt, vorzugsweise zwischen 70 und 300 m²/g, besonders bevorzugt zwischen 80 und 250 m²/g und **dadurch dass**
- die Agglomerate ein V_{80Å} größer oder gleich 20 ml/100g aufweisen, vorzugsweise größer oder gleich 25 ml/100g, besonders bevorzugt größer oder gleich 30 ml/100g, optimalerweise größer oder gleich 35 ml/100g.

2. Verfahren nach Anspruch 1, **dadurch gekennzeichnet, dass** die Agglomerate ein V_{400Å} größer oder gleich 10 ml/100g aufweisen, vorzugsweise größer oder gleich 15 ml/100g, besonders bevorzugt größer oder gleich 20 ml/100g.

3. Verfahren nach Anspruch 1 oder 2, **dadurch gekennzeichnet, dass** die Agglomerate ein V_{37Å} größer oder gleich 45 ml/100g aufweisen, vorzugsweise größer oder gleich 55 ml/100g.

4. Verfahren nach einem der Ansprüche 1 bis 3, **dadurch gekennzeichnet, dass** die Agglomerate eine oder mehrere dotierende Verbindungen umfassen, ausgewählt aus Alkalimetall-, Erdalkalimetallverbindungen und Verbindungen seltener Erden, mit einem maximalen Gehalt von 20 %, vorzugsweise weniger als 10 %.

5. Verfahren nach einem der Ansprüche 1 bis 4, **dadurch gekennzeichnet, dass** die Agglomerate in Form von Kügelchen vorliegen.

6. Verfahren nach Anspruch 5, **dadurch gekennzeichnet, dass** die Kügelchen einen Durchmesser aufweisen, der kleiner oder gleich 8 mm ist, vorzugsweise zwischen 1 und 5 mm.

7. Verfahren nach einem der Ansprüche 1 bis 4, **dadurch gekennzeichnet, dass** die Agglomerate in Form von extrudierten Materialien vorliegen.

8. Verfahren nach Anspruch 7, **dadurch gekennzeichnet, dass** die extrudierten Materialien eine zylindrische Form aufweisen.

9. Verfahren nach Anspruch 7, **dadurch gekennzeichnet, dass** die extrudierten Materialien eine mehrlappige Form aufweisen.

10. Verfahren nach einem der Ansprüche 7 bis 9, **dadurch gekennzeichnet, dass** die extrudierten Materialien einen Querschnittsinnendurchmesser aufweisen, der kleiner oder gleich 4 mm ist.

11. Verfahren nach einem der Ansprüche 1 bis 4, **dadurch gekennzeichnet, dass** die Aluminiumoxid-Agglomerate in Form von Pulver vorliegen.

12. Verfahren nach einem der Ansprüche 1 bis 11, **dadurch gekennzeichnet, dass** das Abwasser oder Abgas ein Medium auf Dichlorethylenbasis darstellt und **dadurch**, **dass** das Metallhalogenid ein Eisenchlorid ist.
